# EUROPÄISCHE PATENTSCHRIFT

(11) **EP 0 483 692 B1**
(45) Veröffentlichungstag und Bekanntmachung des Hinweises auf die Patenterteilung: **07.06.1995**
(21) Anmeldenummer: 91118266.5
(22) Anmeldetag: 25.10.1991
(51) Int. Cl.: A61F 13/15

(54) **Wegwerfkleidungsstück des Höschentyps**
Disposable garment such as pants or the like
Vêtement à jeter du type culotte

(30) Priorität: 25.10.1990 JP 111729/90
(43) Veröffentlichungstag der Anmeldung: 06.05.1992
(73) Patentinhaber: UNI-CHARM CORPORATION, Kawanoe-shi Ehime-ken (JP)
(72) Erfinder: Nomura, Hironori, Iyomishima-shi, Ehime-ken (JP); Igaue, Takamitsu, Kawanoe-shi, Ehime-ken (JP); Tanji, Hiroyuki, Kawanoe-shi, Ehime-ken (JP); Ohnishi, Hirofumi, Iyomishima-shi, Ehime-ken (JP); Sasaki, Tohru, Kanada-cho, Kawanoe-shi (JP)
(74) Vertreter: Sperling, Rüdiger, Dipl.-Ing.

(56) Entgegenhaltungen:
- EP-A- 0 187 727
- EP-A- 0 300 615
- EP-A- 0 412 549

## Beschreibung

Die vorliegende Erfindung betrifft ein Wegwerfkleidungsstück des Höschentyps und insbesondere ein solches Kleidungsstück, dessen einander gegenüberliegende Seitenränder von Verbindungsstreifen begrenzt sind, entlang denen der Vorder- und Hinterteil mit Unterbrechungen miteinander verbunden werden.

Wegwerfkleidungsstücke wie etwa Erziehungshöschen oder eine Windel umfassen im wesentlichen eine flüssigkeitsdurchlässige obere Lage, eine flüssigkeitsundurchlässige untere Lage und oftmals, insbesondere im Fall einer Windel, einen zwischen dieser oberen und unteren Lage eingelegten Absorptionskern. Neben diesen grundsätzlichen Bestandteilen enthält ein derartiges Kleidungsstück nach dem Stand der Technik gewöhnlich elastische Elemente, die in Verbindung mit Hüft- und Beinöffnungen jeweils vorgesehen sind, um die Paßform beim Tragen und das Verhindern des Austritts von Körperflüssigkeiten zu gewährleisten. Bezüglich der Ausführungen eines derartigen Produktes ist es andererseits, neben einer sogenannten offenen Ausführung, die vielfach benutzt wurde, aufgrund ihrer einfachen Verwendbarkeit die höschenartige Ausführung, die die Aufmerksamkeit der Verbraucher auf sich zog. Die höschenartige Ausführung ist insbesondere deshalb vorteilhaft, da z.B. eine gute Paßfähigkeit beim Tragen erzielt wird, Probleme mit dem an der Hüft- und/oder den Beinöffnungen auftretenden Austreten von Körperflüssigkeiten im wesentlichen verhindert werden und der Trage- bzw. Anlegevorgang sehr einfach ist.

### BESCHREIBUNG DER ERFINDUNG

Die vorliegende Erfindung richtet sich auf ein Wegwerfkleidungsstück des Höschentyps, das wenigstens eine flüssigkeitsdurchlässige obere Lage, eine flüssigkeitsundurchlässige untere Lage und einer Hüftöffnung und Beinöffnungen jeweils zugeordnete elastische Elemente umfaßt, wobei die gegenüberliegenden Seitenränder dieses Kleidungsstücks von Verbindungsstreifen gebildet werden, entlang welcher der Vorder- und Hinterteil miteinander verbunden werden. Gemäß bekannter Techniken wurde dieses Verbinden durch das Verbinden von Vorderteil und Hinterteil entlang einzelnen oder mehreren durchgehenden Verbindungsstreifen, die jeweils von der Hüftöffnung zur zugehörigen Beinöffnung verlaufen, erzielt. Es wurde jedoch festgestellt, daß derartige Verbindungsstreifen notwendigerweise eine gewünschte Dehnbarkeit in Längsrichtung des Kleidungsstücks, wie etwa einer Wegwerfwindel des Höschentyps, behindern. Die Lösung dieses Problems ist von größter Wichtigkeit, insbesondere wenn die Windel des Höschentyps aus dehnbaren Werkstoffen aufgebaut ist, um die Dehnbarkeit dieser Windel zu verbessern. Darüber hinaus führen diese Verbindungsstreifen oftmals zu einem Aufbau, der beispielsweise die Windeln des Höschentyps luftdicht abschließt und es schwierig macht, die besonders bei Windeln des Höschentyps erforderliche gute Luftdurchlässigkeit zu erreichen. Darüber hinaus ist das Verfahren zum Bilden der Verbindungsstreifen entlang der gegenüberliegenden Seitenränder, die elastische Elemente enthalten, wesentlich komplizierter als in einem Fall, in dem die einander gegenüberliegenden Seitenränder keine elastischen Elemente enthalten.

Demgemäß ist es eine wesentliche Aufgabe der vorliegenden Erfindung, ein Wegwerfkleidungsstück des Höschentyps aufzuzeigen, das eine neuartige Verbindungsstruktur an den einander gegenüberliegenden Seitenrändern aufweist, um damit die verschiedenen, vorstehend erläuterten Probleme zu lösen.

Die vorstehend aufgeführte Aufgabe wird gemäß vorliegender Erfindung durch ein Wegwerfkleidungsstück des Höschentyps gelöst, das eine flüssigkeitsdurchlässige obere Lage und eine flüssigkeitsundurchlässige untere Lage enthält, wobei Vorder- und Hinterteil entlang der einander gegenüberliegenden Seitenränder des Kleidungsstücks miteinander verbunden werden, und jeweils einer Hüftöffnung und den entsprechenden Beinöffnungen zugeordnete elastische Elemente vorhanden sind, dadurch gekennzeichnet, daß die elastischen Elemente zwischen der oberen und unteren Lage eingelegt sind, in Längsrichtung gegenüberliegende Enden der jeweiligen elastischen Elemente an den einander gegenüberliegenden Seitenrändern des Vorderteils und des Hinterteils angeordnet sind und die einander gegenüberliegenden Seitenränder des Vorder- und Hinterteils mit Unterbrechungen miteinander verbunden sind, so daß die einander in Längsrichtung gegenüberliegenden Enden des elastischen Elemente nicht erfaßt werden.

Gemäß einer bevorzugten Ausführungsform sind die gegenüberliegenden Seitenränder mit Grifflaschen versehen, die sich vom Kleidungsstück nach außen erstrecken.

Gemäß einer weiteren bevorzugten Ausführungsform umfassen sowohl die flüssigkeitsdurchlässige obere Lage als auch die flüssigkeitsundurchlässige untere Lage elastisch dehnbare Schichten.

Gemäß einer weiteren bevorzugten Ausführungsform umfaßt jedes der elastischen Elemente mehrere Einzelelemente.

Das Wegwerfkleidungsstück des Höschentyps wie z.B. Wegwerfhöschenwindeln, die gemäß der vorliegenden Erfindung aufgebaut sind, ist an seinen seitlich gegenüberliegenden Seitenrändern durch Verbindungsstreifen begrenzt, entlang welchen der Vorderteil und der Hinterteil mit Unterbrechungen miteinander verbunden werden, so daß jeder der seitlich gegenüberliegenden Seitenränder wechselweise Verbindungsbereiche und nicht verbundene Bereiche aufweist. Die nicht verbundenen Bereiche verbleiben dehnbar, tragen zur Verbesserung der Längsdehnbarkeit des Wegwerfkleidungsstücks des Höschentyps einerseits bei und bilden seitliche Öffnungen, die zur Verbindung des Inneren mit dem Äußeren des Wegwerfkleidungsstücks des Höschentyps dienen und somit dem Wegwerfkleidungsstück des Höschentyps eine verbesserte Luftdurchlässigkeit bzw. einen Belüftungseffekt verleihen.

Des weiteren ist eine besondere Anordnung vorgesehen, daß die Verbindung so ausgeführt ist, daß die in Längsrichtung gegenüberliegenden Enden der elastischen Elemente nicht betroffen sind, was nicht nur den zum Verbinden erforderlichen Arbeitsaufwand verringert, sondern es auch unnötig macht, eine spezielle Vorgehensweise anzuwenden, um die elastischen Elemente in der Weise anzuordnen, daß in Längsrichtung gegenüberliegenden Enden der elastischen Elemente nicht während des Verbindens an den einander seitlich gegenüberliegenden Seitenrändern des Vorder- und Hinterteils zu liegen kommen.

Darüber hinaus kann das einmal einem Träger angelegte Kleidungsstück des Höschentyps ohne weiteres vom Träger entfernt werden, indem der Vorderteil und Hinterteil entlang der Verbindungsstreifen in vorteilhafter Weise auseinandergezogen werden, wobei nur das Gefühl eines gemäßigten Widerstandes gegen das Auseinanderziehen auftritt, wie es in etwa auch erfahren wird, wenn ein Reißverschluß geöffnet wird. Derartiges Abziehen ist weiter durch das Vorsehen von Grifflaschen an den seitlich gegenüberliegenden Seitenrändern des Kleidungsstücks erleichert.

Bei der Verwendung der flüssigkeitsdurchlässigen Schicht als obere Lage bzw. der flüssigkeitsundurchlässigen Schicht als untere Lage wirkt sich die punktweise bzw. mit Unterbrechungen vorgesehene Verbindung niemals hindernd auf die Längsdehnbarkeit des Kleidungsstücks aus und stellt sicher, daß eine gute Luftdurchlässigkeit bzw. ein Belüftungseffekt des Kleidungsstücks aufrechterhalten bleibt.

Nachfolgend wird die Erfindung unter Bezug auf die in den beiliegenden Zeichnungen erläuterten Ausführungsformen beschrieben.

### KURZE BESCHREIBUNG DER ZEICHNUNGEN

- Fig. 1: zeigt eine perspektivische Darstellung einer Windel als erster Ausführungsform der Erfindung;
- Fig. 2: zeigt eine auseinandergezogene perspektivische Darstellung der in Fig.1 gezeigten Windel;
- Fig. 3: zeigt eine vergrößerte Detaildarstellung der in Fig.1 gezeigten Windel;
- Fig. 4 u.5: sind vergrößerte perspektivische Teildarstellungen einer weiteren Ausführungsform der Erfindung; und
- Fig. 6: zeigt eine perspektivische Darstellung einer unteren Lage einer weiteren Ausführungsform der Erfindung.

### BEVORZUGTE AUSFÜHRUNGSFORMEN DER ERFINDUNG

### Ausführungsform 1

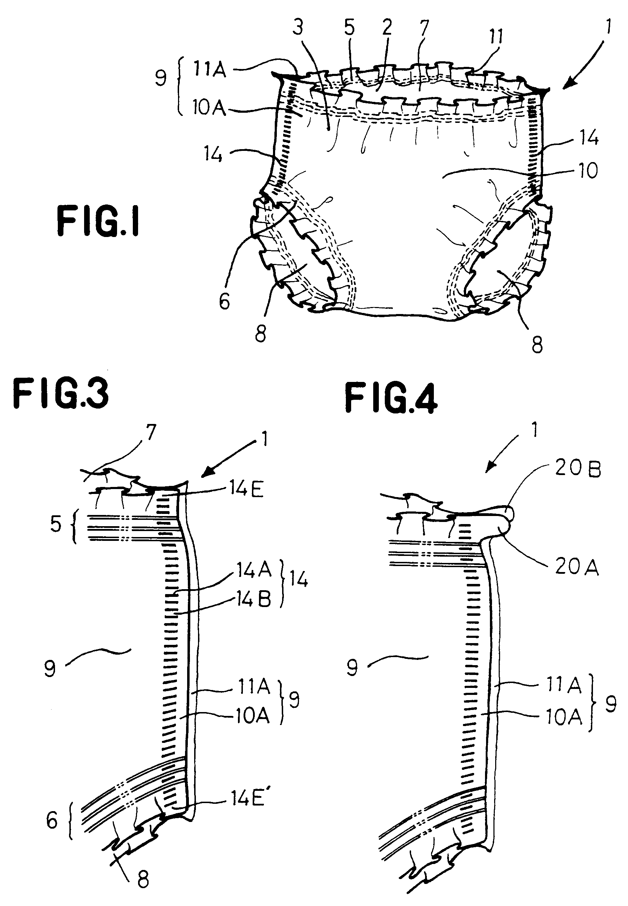

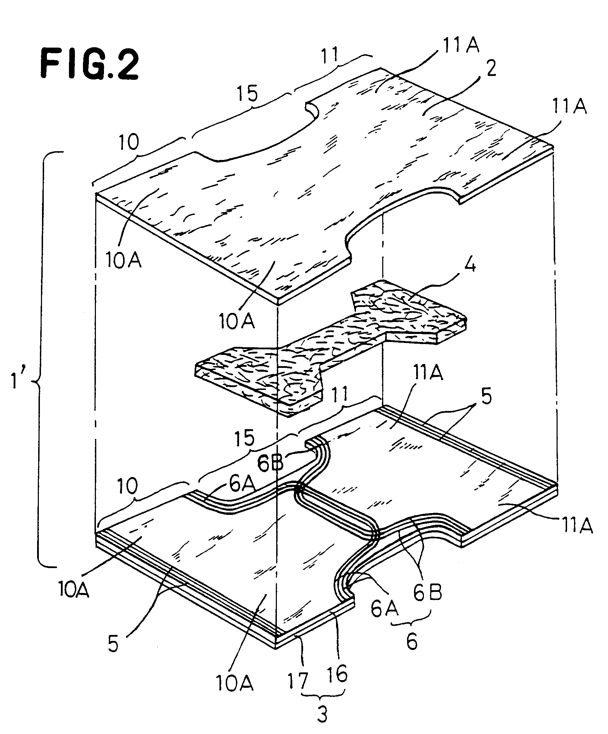

Bezugnehmend auf Fig.1, die eine perspektivische Darstellung eines Wegwerfwindelhöschens als spezielle Ausführungsform der Erfindung zeigt, umfaßt die Windel 1 eine flüssigkeitsdurchlässige obere Lage 2, eine flüssigkeitsundurchlässige untere Lage 3, einen Absorptionskern 4 (s.Fig.2), Gummifäden 5, d.h. elastische Elemente, die einer Hüftöffnung 7 zugeordnet sind, und Gummifäden 6, d.h. elastische Elemente, die Beinöffnungen 8 zugeordnet sind. Die jeweiligen Umfangsränder der Hüftöffnung 7 und der Beinöffnungen 8 bilden unter der Kontraktion der zugeordneten Gummifäden 5 und 6 Raffungsfalten. Ein Vorderteil 10 und ein Hinterteil 11 sind an den einander seitlich gegenüberliegenden Seitenrändern 9 miteinander verschweißt, d.h. an den jeweiligen einander seitlich gegenüberliegenden Seitenrändern 10A, 11A, und bilden so die Windel 1 des Höschentyps. Hier sei angemerkt, daß die Windel 1 im allgemeinen im wesentlichen in seitlicher Richtung symmetrisch ist. Auch sollte klar sein, daß der in dieser speziellen Ausführungsform integrierte Absorptionskern 4 nicht notwendigerweise zur Ausführung der vorliegenden Erfindung erforderlich ist.

In Fig.2 ist die in Fig.1 dargestellte, auf einem kontinuierlichen Fertigungsband hergestellte Windel 1 in einer perspektivisch auseinandergezogenen Darstellung gezeigt. In Längsrichtung gesehen umfaßt die Windel den Vorderteil 10, den Schrittbereich 15 und den Hinterteil 11, und in Richtung ihrer Stärke gesehen, umfaßt die Windel 1 die obere Lage 2, den Absorptionskern 4 und die untere Lage 3. Die obere Lage 2 ist aus elastisch dehnbarem Vliesstoff hergestellt, der wiederum durch ein Verschlingungsverfahren unter Wasserstrahlen und anschließender Warmkräuselung von thermoplastischen Fasern hergestellt wird, und der Absorptionskern 4 ist vorzugsweise aus flockiger Pulpe geformt, der 5 bis 15 Gew.-% eines geeigneten wasserabsorptiven Polymers beigemischt sind. Die untere Lage 3 ist aus zwei Schichten hergestellt, die punktweise mittels Heißschmelzkleber miteinander verschweißt sind, wobei eine dieser Schichten eine elastisch dehnbare, flüssigkeitsundurchlässige Folie 16 aus thermoplastischem Polyethylenelastomer und die andere Schicht einen dehnbaren Vliesstoff 17 umfaßt, der durch das Verschlingungsverfahren und die nachfolgende Warmkräuselung von thermoplastischen Fasern hergestellt ist. Die flüssigkeitsundurchlässige Folie 16 ist der oberen Lage 2 gegenüberliegend angeordnet, wobei der Absorptionskern 4 dazwischengelegt ist, und der Vliesstoff 17 bildet die äußere Oberfläche der Windel 1. Selbstverständlich dient die flüssigkeitsundurchlässige Schicht 16 dazu, zu verhindern, daß die untere Lage 3 durchlässig für Flüssigkeiten ist und der Vliesstoff 17 dient dazu, der Windel an ihrer äußeren Oberfläche eine angenehm zu berührende Schicht zu geben. Die punktweise Verbindung von flüssigkeitsundurchlässiger Folie 16 und dem Vliesstoff 17 erlaubt es, die Festigkeit der unteren Lage 3 zu verbessern, ohne daß ihre Dehnbarkeit beeinträchtig wird. Die untere Lage 3 weist auf der flüssigkeitsundurchlässigen Schicht 16 die Gummifäden 5, d.h. die der Hüftöffnung zugehörigen elastischen Elemente sowie die Gummifäden 6, d.h. die den Beinöffnungen zugehörigen elastischen Elemente auf; genauer gesagt, Gummifäden 6A und 6B, die den jeweiligen Beinöffnungen zugeordnet sind. Jedes dieser elastischen Elemente kann aus mehreren einzelnen elastischen Fäden oder elastischen Einzelelementen bestehen, die im gespannten Zustand punktweise mit der flüssigkeitsundurchlässigen Folie 16 mittels Heißschmelzkleber verbunden sind. Es ist anzumerken, daß der Teilabschnitt der Gummifäden 6A, 6B, der zwischen ihren beiden Kreuzungspunkten im Schrittbereich 15 verläuft, im wesentlichen nicht gespannt ist. Die Gummifäden 5, 6A, 6B, d.h. die elastischen Elemente können aus Werkstoffen gefertigt sein, die, wie auch die Anordnungen, auf dem Gebiet der Wegwerfwindeln bekannt sind, und beispielsweise fadenartige oder bandartige elastische Elemente. Diese Gummifäden 5, 6A, 6B werden entlang der Laufrichtung auf einem kontinuierlichen Produktionsband der Windel 1 kontinuierlich zugeführt, d.h. quer zur Windel 1, so daß ihre gegenüberliegend abgeschnittenen Enden auf die Seitenränder der unteren Lage gebracht werden, die den seitlich gegenüberliegenden Seitenrändern 9 entsprechen (s.Fig.3). Die Anordnung der Gummifäden 6A, 6B auf der flüssigkeitsundurchlässigen Folie 16 erfolgt dergestalt, daß diese Gummifäden 6A, 6B paarweise ausgelegt werden, um die jeweiligen elastischen Elemente (Gummifäden 6 in Fig.1) für die jeweiligen, im wesentlichen kreisförmigen Beinöffnungen 8 zu bilden. Einzelheiten dieser Anordnung werden nicht weiter erläutert.

Die obere Lage 2 und die untere Lage 3 sind in ihrem äußeren Umfang identisch und haben außerhalb des Absorptionskernes 4 verlaufende Überstände, so daß die beiden Lagen 2 und 3 im Bereich dieser Überstände punktweise miteinander verschweißt werden. Ein somit erhaltener Schichtkörper 1' (s.Fig.2) wird entlang seiner in Längsrichtung gesehenen Mittellinie zusammengefaltet, wobei die obere Lage 2 nach innen weist und der Vorderteil 10 auf den Hinterteil 11 aufgelegt wird. Anschließend werden der Vorderteil 10 und der Hinterteil 11 mittels Schweißstreifen 14 entlang den jeweiligen einander gegenüberliegenden Seitenrändern 10A und 11A miteinander verschweißt, um so die Windel 1 des Höschentyps zu bilden. Während dieses Schichtvorganges liegen die Gummifäden 5, 6A am Vorderteil 10 im wesentlichen gegenüber den Gummifäden 5, 6B am Hinterteil 11 und bilden so die im wesentlichen endlosen elastischen Elemente, die der Hüftöffnung 7 bzw. den Beinöffnungen 8 zugeordnet sind.

In Fig.3 ist einer der einander seitlich gegenüberliegenden Seitenränder 9 einschließlich des Schweißstreifens 14 in vergrößertem Maßstab ausrißweise dargestellt. Jeder der einander seitlich gegenüberliegenden Seitenränder 9 umfaßt die Seitenränder 10A, 11A des Vorderteils 10 bzw. des Hinterteils 11. Der Schweißstreifen 14 umfaßt verschweißte Abschnitte 14A, in welchen die Werkstoffe des Vorderteils 10 und des Hinterteils 11 mittels einer Ultraschallschweißtechnik miteinander verschweißt sind, und nicht verschweißte Abschnitte 14B, in welchen das Ultraschallschweißen nicht stattgefunden hat, wobei sich die verschweißten Abschnitte 14A und die nicht verschweißten Abschnitte 14B abwechseln. Der Schweißstreifen 14 verläuft von der Hüftöffnung 7 zur zugehörigen Beinöffnung 8. Ein größerer Teil jedes Schweißstreifens 14 wird von einem abwechselnden Muster in der Weise gebildet, daß die verschweißten Abschnitte 14A von annähernd 5 x 1 mm (Breite x Höhe) und die nicht verschweißten Abschnitte 14B von annähernd 5 x 1,5 mm (Breite x Höhe) einander abwechseln. Wie jedoch aus Fig.3 ersichtlich ist, sind die verschweißten Abschnitte 14A in der Nähe der elastischen Elemente für die jeweiligen Beinöffnungen schräg gestellt, so daß sie im wesentlichen parallel zu den Gummifäden 6A bzw. 6B verlaufen. Die Gummifäden 5, 6A und 6B sind von dem entlang den Schweißstreifen 14 ausgeführten Ultraschallschweißvorgängen nicht betroffen, d.h. sie liegen in nicht verschweißten Abschnitten 14B. Das obere Ende und/oder das untere Ende jedes Schweißstreifens 14 kann mit nicht verschweißten Zonen 14E und 14E' versehen sein, die geringfügig größer sind als die übrigen Abschnitte 14B, so daß Vorder- und Hinterteil leicht entlang den Schweißlinien 14 auseinanderziehbar sind, indem diese nicht verschweißten Abschnitte 14E, 14E' gehalten werden. Wenn jeder der verschweißten Abschnitte 14A so dimensioniert ist, daß er eine Breite von 0,5 bis 20 mm, vorzugsweise 1 bis 15 mm, gemessen in seitlicher Richtung der Windel, eine Höhe von 0,5 bis 30 mm, vorzugsweise 1 bis 15 mm in vertikaler Richtung der Windel 1 hat und daß er 40 bis 90 %, vorzugsweise 50 bis 80 % der Gesamtfläche jedes Schweißstreifens 14, der von der Hüftöffnung 7 zur zugehörigen Beinöffnung 8 verläuft, einnimmt, so können die verschweißten Abschnitte 14A jegliche Anordnung und jegliches Wiederholungsmuster haben, solange die elastischen Elemente frei von der Ultraschallverschweißung sind. Nehmen jedoch die verschweißten Abschnitte 14A weniger als 40 % der Gesamtfläche jedes Schweißstreifens 14 ein, so verursacht das möglicherweise Probleme, wie etwa mangelhafte Festigkeit der Schweißstreifen 14 und Austreten von Körperflüssigkeiten. Nehmen im Gegensatz dazu die verschweißten Abschnitte 14A 90 % oder mehr ein, so kann dies die Dehnbarkeit sowie die Luftdurchlässigkeit wesentlich beeinträchtigen.

Die in vorstehend beschriebener Weise hergestellte Windel 1 ist in erfinderischer Weise mit Dehnbarkeit in Längsrichtung der Windel 1 versehen, die durch das Vorhandensein von nicht verschweißten Abschnitten 14B bedingt ist, womit sie mit einer für die elastisch dehnbare Windel 1 erwünschten guten Paßfähigkeit versehen ist. In den nicht verschweißten Abschnitten 14B findet ein Luftaustausch zwischen im Inneren und dem Äußeren der Windel 1 statt, womit eine gute Luftdurchlässigkeit oder Belüftung erreicht wird, obwohl die flüssigkeitsundurchlässige Folie 16 verwendet wird. Im Verlauf des Ultraschallschweißvorganges bleiben die Bereiche der einander seitlich gegenüberliegenden Seitenränder 9, in denen die Gummifäden 5, 6A und 6B mit örtlich unterschiedlicher Stärke wie auch Härte vorhanden sind, frei vom Einfluß der Ultraschallschweißbehandlung, womit die Schweißmaschine oder das Schweißgerät entsprechend konstruiert werden kann, genauer gesagt, so konstruiert, daß die entsprechenden Abschnitte nicht verschweißt werden, womit der gewünschte Verschweißungseffekt in bequemer Weise erzielbar ist.

Zum Abnehmen der Höschenwindeln 1 vom Träger können der Vorderteil 10 und der Hinterteil 11 an den Abschnitten ihrer Seitenränder 9 gehalten werden, die sich außerhalb der Schweißlinie erstrecken, und voneinander weggezogen werden. Auf diese Weise können der Vorderteil 10 und der Hinterteil 11 mit Leichtigkeit voneinander weggezogen werden. Darüber hinaus ergibt das sich wiederholende Muster der verschweißten Abschnitte 14A und der nicht verschweißten Abschnitte 14B das Gefühl eines Abziehwiderstandes, der dem Gefühl beim Bedienen eines Reißverschlusses entspricht.

In Fig.4 ist als Beispiel eine Anordnung dargestellt, bei der der Vorderteil 10 und der Hinterteil 11 an ihren Seitenrändern 10A und 11A mit Grifflaschen 20A bzw. 20B versehen sind, die von den jeweiligen Seitenrändern 10A und 11A nach außen verlaufen. Diese Grifflaschen 20A und 20B können in einem Stück mit der oberen Lage 2 und/oder der unteren Lage 3 oder getrennt von diesen Lagen 2 und 3 geformt sein.

### Ausführungsform 2

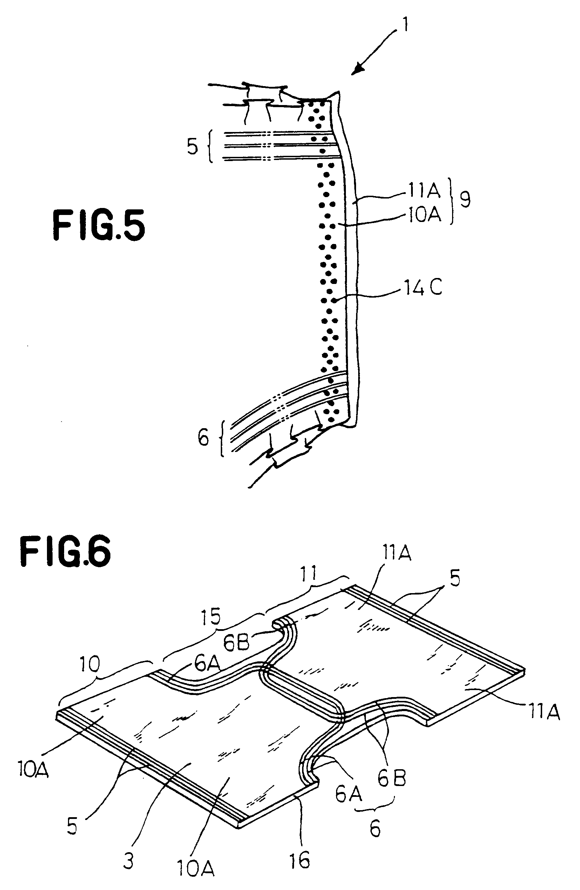

In Fig.5 ist die Ausführungsform 2 der Erfindung dargestellt, die im wesentlichen der vorstehend als Ausführungsform 1 beschriebenen Windel ähnlich ist, mit der Ausnahme, daß ein Punkte 14C umfassender Verbindungsstreifen 14 eine seitlich gesehene Breite von annähernd 10 mm aufweist und, anstelle des ultraschallgeschweißten Streifens 14, aus Heißschmelzkleber bestehende Punkte 14C, die jeweils einen Durchmesser von 2 bis 4 mm haben, aufweist und daß die Klebepunkte annähernd 60% der Gesamtfläche des Verbindungsstreifens 14 einnehmen. Auch bei dieser Ausführungsform sind die Klebepunkte 14C so aufgebracht, daß die Gummifäden 5, 6A und 6B frei bleiben.

Die gemäß dieser Ausführungsform erhaltene Windel 1 bietet nicht nur die gewünschte Dehnbarkeit aufgrund des Vorhandenseins von nicht verbundenen Abschnitten zwischen den Klebepunkten 14C, sondern auch eine gute Luftdurchlässigkeit und andere Effekte, wie sie auch die Ausführungsform 1 bietet.

### Ausführungsform 3

Fig. 6 zeigt eine perspektivische Darstellung der unteren Lage 3 in Ausführungsform 3 der Erfindung, die der Windel 1 in Ausführungsform 1 ähnlich ist, mit der Ausnahme, daß die untere Lage in angepaßter Ausführung vorgesehen ist. Gemäß dieser Ausführungsform umfaßt, ähnlich wie bei Ausführungsform 1, die obere Lage 2 den elastisch dehnbaren Vliesstoff, der aus warmgekräuselten thermoplastischen Fasern, die unter Behandlung mit Hochdruckwasserstrahlen umeinandergeschlungen, d.h. verschlungen wurden, und auch der Absorptionskern 4 ist mit dem in Ausführungsform 1 verwendeten identisch. Die untere Lage 3 jedoch, wie in Fig.6 gezeigt, umfaßt eine einlagige, elastisch dehnbare flüssigkeitsundurchlässige Folie 16, die aus thermoplastischen Polyethylenelastomer hergestellt ist. Diese geänderte Anordnung erlaubt im Vergleich zu den vorherigen Ausführungsformen eine effektive Reduzierung der Herstellungskosten.

## Patentansprüche

1. Wegwerfkleidungsstück (1) des Höschentyps mit einer flüssigkeitsdurchlässigen oberen Lage (2) und einer flüssigkeitsundurchlässigen unteren Lage (3), wobei Vorder- und Hinterteil (10,11) entlang ihren einander seitlich gegenüberliegenden Seitenrändern (9) zusammengefügt sind und einer Hüftöffnung (7) und Beinöffnungen (8) jeweils zugeordnete elastische Elemente (5,6) vorgesehen sind,
**dadurch gekennzeichnet,**
daß die elastischen Elemente (5,6) zwischen der oberen und der unteren Lage (2,3) eingelegt sind, in Längsrichtung gegenüberliegende Enden der jeweiligen elastischen Elemente (5,6) an den einander seitlich gegenüberliegenden Seitenrändern (9) des Vorder- und Hinterteils (10,11) angeordnet sind und die einander seitlich gegenüberliegenden Seitenränder (9) des Vorder- und Hinterteils (10,11) mit Unterbrechungen (14B) so miteinander verbunden sind, daß die einander in Längsrichtung gegenüberliegenden Enden der elastischen Elemente (5,6) ausgespart sind.

2. Kleidungsstück nach Anspruch 1, wobei die einander seitlich gegenüberliegenden Seitenränder (9) mit Grifflaschen (20A,20B) versehen sind, die sich vom Kleidungsstück (1) nach außen erstrecken.

3. Kleidungsstück nach Anspruch 1, wobei sowohl die flüssigkeitsdurchlässige obere Lage (2) als auch die flüssigkeitsundurchlässige untere Lage (3) eine oder mehrere elastisch dehnbare Schichten (16,17) umfassen.

4. Kleidungsstück nach Anspruch 1, wobei jedes der elastischen Elemente (5,6) mehrere elastische Einzelelemente umfaßt.

## Claims

1. Disposable garment (1) of the pants type, with a liquid-permeable upper layer (2) and a liquid-impermeable lower layer (3), the forward and rear portions (10, 11) being combined along their mutually laterally adjacent lateral ledges (9), and resilient members (5, 6) being provided associated with a hip opening (7) and leg openings (8), characterised in that the resilient members (5, 6) are inset between the upper and lower layers (2, 3), longitudinally mutually oppositely-lying ends of the respective resilient members (5, 6) being disposed at the mutually laterally oppositely-lying lateral edges (9) of the forward and rear portions (10, 11), and the mutually laterally oppositely-lying lateral edges (9) of the forward and rear portions (10, 11) being connected together with interruptions (148) in such a way that the longitudinally mutually oppositely-lying ends of the resilient members (5, 6) contain apertures.

2. Garment according to claim 1, wherein the mutually laterally oppositely-lying lateral edges (9) are provided with tab handles (20A, 20B) which extend outwards from the garment (1).

3. Garment according to claim 1, wherein both the liquid-permeable upper layer (2) and the liquid-impermeable lower layer (3) include one or a plurality of elastically expandable layers (16, 17).

4. Garment according to claim 1, wherein each of the resilient members (5, 6) includes a plurality of elastic individual elements.

## Revendications

1. Vêtement jetable (1) du genre petite-culotte, comportant une couche supérieure perméable aux liquides (2), et une couche inférieure imperméable aux liquides (3), les parties avant et arrière (10, 11) de ces couches étant assemblées le long de leurs bords latéraux (9) en opposition latérale mutuelle, et des éléments élastiques (5, 6) correspondant, respectivement, à une ouverture de passage des hanches (7) et à des ouvertures de passage des jambes (8) étant prévus, caractérisé en ce que les éléments élastiques (5, 6) sont insérés entre les couches supérieure et inférieure (2, 3), en ce que des extrémités des éléments élastiques respectifs (5, 6), opposées dans le sens de la longueur, sont placées sur les bords latéraux (9) en opposition latérale mutuelle des parties avant et arrière (10, 11), et en ce que les bords latéraux (9) en opposition latérale mutuelle des parties avant et arrière (10, 11) sont assemblés entre eux avec des interruptions (14B), de telle façon que les extrémités des éléments élastiques (5, 6), opposées dans le sens de la longueur, sont laissées libres.

2. Vêtement selon la revendication 1, dans lequel les bords latéraux (9) en opposition latérale mutuelle, sont pourvus de languettes de préhension (20A, 20B) qui s'étendent vers l'extérieur, en partant du vêtement (1).

3. Vêtement selon la revendication 1, dans lequel aussi bien la couche supérieure (2) perméable aux liquides, que la couche inférieure (3) imperméable aux liquides, comportent une ou plusieurs couches élastiques extensibles (16, 17).

4. Vêtement selon la revendication 1, dans lequel chacun des éléments élastiques (5, 6) comporte plusieurs éléments élastiques individuels.
